Europäisches Patentamt

European Patent Office

Office européen des brevets

(19)

(11) EP 1 114 814 A2

(12) EUROPEAN PATENT APPLICATION

(43) Date of publication:
11.07.2001 Bulletin 2001/28

(51) Int Cl.⁷: **C07C 51/487**, C07C 51/493,
C07C 53/00, C07C 53/08

(21) Application number: 00127663.3

(22) Date of filing: 18.12.2000

(84) Designated Contracting States:
**AT BE CH CY DE DK ES FI FR GB GR IE IT LI LU MC NL PT SE TR**
Designated Extension States:
**AL LT LV MK RO SI**

(30) Priority: **29.12.1999 US 173472 P**

(71) Applicant: **Haldor Topsoe A/S
2800 Kgs. Lyngby (DK)**

(72) Inventor: **Schiodt, Niels Christian
2700 Bronshoj (DK)**

(54) **Method for the reduction of iodine compounds from a process stream**

(57) A method for the reduction in iodine content in a mixture, particularly in a mixture of carboxylic acids and/or carboxylic anhydrides with alkyl iodides and/or hydriodic acid, comprises contacting the organic medium in the vapour phase at elevated temperature with a metal salt dispersed on activated charcoal. The metal salt may be a carboxylate salt, a carbonate, an oxide, a hydroxide and any other salt, which may react with the organic medium to give a metal carboxylate salt. The metal may be a main group element, a lanthanide, an actinide, zinc and copper. This process converts alkyl iodide compounds converted to their corresponding carboxylate esters, while the iodine is bound in the purification mass as inorganic iodide.

## Description

The present invention relates to a process for the removal of iodine from iodine-containing compounds, e.g. alkyl iodides and the like, which are contained in a mixture with other compounds, more specifically with carboxylic acids and/or carboxylic anhydrides. In particular, the present invention is suited for the purification of acetic acid and/ or acetic anhydride prepared by the rhodium or iridium-catalysed, methyl iodide promoted carbonylation of methanol and/or dimethyl ether and/or methyl acetate.

Within industrial acetic acid production, it is well known that the final product in order to meet the demands of the purchasers in many cases must be extremely low (less than 6 parts per billion weight (ppb)) in iodide. Approximately 50% of the global acetic acid production are used for vinyl acetate manufacture in a catalytic process. The catalyst for this process is severely damaged by iodide even in very low concentrations. Iodide in acetic acid stems from the use of methyl iodide and/or hydriodic acid as co-catalyst in the carbonylation of methanol and/or dimethyl ether and/or methyl acetate. Most of the iodine is present in the form of methyl iodide in the crude acetic acid, while smaller amounts are present as hydriodic acid and higher alkyl iodides. Methyl iodide is easily separated by distillation and recycled to the reactor zone. Most of the hydriodic acid may also be recovered. The residual iodides in the product, mainly higher (C2-C8) alkyl iodides are more difficult to remove. Hexyl iodide is often mentioned since this compound is particularly troublesome to remove by distillation due to its boiling point being very close to that of acetic acid. The concentration of higher alkyl iodides is typically of the order of 100-1000 ppb (0.1-1 ppm).

Several methods for the purification of crude carboxylic acids and/or acetic acid and/or derivatives thereof have been described. Most of these methods, however, concern purification in the liquid phase. Thus, several patents teach the use of silver, palladium, mercury and/or rhodium-exchanged resins (U.S. Patent Nos. 4,975,155, 5,220,058, 5,227,524, 5,300,685 and 5,801,279) for reducing the level of iodine in carboxylate streams in the liquid phase. Purification by the addition of oxidising agents is taught e.g. by U.S. Patent No. 5,387,713 describing the use of hydrogen peroxide in the liquid phase and by U.S. Patent No. 5,155,265, which describes the use of ozone as purifying agent in the liquid phase. More related to the present invention is U.S. Patent No. 4,246,195, which teaches the use of caesium, potassium and sodium acetate for purification of carbonylation products with respect to iodine contaminants. Said patent, however, deals solely with operations in the liquid phase.

It is an object of the present invention to provide a method for reducing the iodine level in a mixture, particularly in a mixture of acetic acid with alkyl iodides and/or hydriodic acid in the vapour phase.

In accordance with the invention, it has surprisingly been discovered that when acetic acid contaminated with alkyl iodides in a concentration from 0.1 to 3000 ppm has been contacted in the vapour phase at elevated temperatures and normal pressure with metal acetate salts dispersed on a special support material, the alkyl iodides have been converted to the corresponding alkyl acetates, while the iodide has become bound as inorganic (non-volatile) iodide in the absorbent. Said special support material is activated charcoal. With other supports such as alumina and silica, there is almost no conversion.

Thus, it was found that when a solution of 258 ppb hexyl iodide in acetic acid was evaporated through a bed of potassium acetate on activated charcoal (bulk volume 7 ml) at a temperature of 189±6°C and at a flow rate of 33±8 g/h, the concentration of hexyl iodide in the re-condensed acetic acid solution had become reduced to below 6 ppb hexyl iodide (below 4 ppb iodine). The hexyl iodide concentration was measured after 20, 40, 60, 120 and 140 minutes on stream and was in all cases found to be below 6-ppb hexyl iodide.

Moreover, by the present invention alkyl iodides are not simply retained on the absorbent due to some chromatographic effect. The following examples will show that alkyl iodides are converted to their corresponding acetate esters that were identified by gas chromatographic analysis. To demonstrate the scope of the present invention, 7 different absorbents were prepared (Examples 1-7). Since the present invention may have a specific application within acetic acid manufacture and purification technology, the performance of some of the absorbents towards an acetic acid solution with a content of hexyl iodide of 100-300 ppb was tested (Examples 8-14). Also, a number of comparative examples with a more concentrated solution of alkyl iodides in acetic acid were conducted (Examples 15-32). These comparative examples were not performed on solutions of hexyl iodide in acetic acid, but rather on mixtures of methyl iodide, butyl iodide and octyl iodide in acetic acid.

## Example 1

Preparation of a potassium on activated charcoal absorbent (KOAc/A.C.).

Potassium acetate (1.96 g, 0.02 mole) was dissolved in de-ionised water (4 ml) and diluted to 7.5 ml with de-ionised water to give a clear, colourless solution. Activated charcoal (10.00 g) in the form of 2.5 mm granules (Merck, QBET surface area 1190 $m^2$/g of which 470 $m^2$/g is microporous surface area) was contacted with the aqueous solution and shaken thoroughly in a closed container until the material appeared dry or almost dry. The material was then dried in an oven at 100°C for 16 hours, after which the weight was recorded to be 13.10 g and the bulk volume to be 28 ml. This material was then divided into four portions of equal weight, thus containing 0.005 mole K each and these portions were stored separately. This procedure was followed in every preparation of absorbent materials.

**Example 2**

An absorbent was prepared as described in Example 1 except that the potassium acetate was replaced with caesium acetate (3.84 g, 0.02 mole).

**Example 3**

An absorbent was prepared as described in Example 1 except that the potassium acetate was replaced with lithium acetate dihydrate (2.04 g, 0.02 mole).

**Example 4**

An absorbent was prepared as described in Example 1 except that the potassium acetate was replaced with zinc acetate dihydrate (4.39 g, 0.02 mole).

**Example 5**

An absorbent was prepared as described in Example 1 except that the potassium acetate was replaced with magnesium acetate tetrahydrate (4.29 g, 0.02 mole).

**Example 6**

An absorbent was prepared as described in Example 1 except that the 10.00 g activated carbon was replaced with 10.00 g calcined alumina (QBET surface area 270 $m^2$/g).

**Example 7**

An absorbent was prepared as described in Example 1 except that the 10.00 g activated carbon was replaced with 10.00 g silica (Merck, 100 mesh).

**Example 8**

A glass reactor of inner diameter 1.0 cm was charged with one portion of the absorbent (bulk volume 7 ml, 0.005 mole potassium acetate) prepared as described in Example 1. The glass reactor was placed in a tube furnace. The lower end of the glass reactor was connected to a well isolated 250 ml round bottomed flask resting in a heating mantle, while the top end was fitted to a Claisen condenser and a receiver flask. The round-bottomed flask was equipped with a thermometer. Through the top of the glass reactor was introduced a thermoelement, which was fixed in the centre of the metal acetate bed during the distillation. The tube furnace was heated to 250°C and the heating mantle was turned on. A solution of n-hexyl iodide (HxI) in acetic acid was introduced into the hot round-bottomed flask by means of a peristaltic pump. The temperature in the acetate bed was measured to be 189°C ± 6°C throughout the experiment. The flow rate was maintained at 33 ± 8 g/h. At regular intervals, the condensate was withdrawn from the receiver flask, the weight of it was recorded in order to measure the flow rate, and the contents of HxI in the sample (thoroughly homogenised) was analysed with GC-MS by a standardised method. The concentration of HxI in the untreated solution was measured to be 258 ppb (258 microgram hexyliodide per kilogram solution). After 20 minutes the purified solution was collected and analysed. The concentration of HxI amounted to 5.4 ppb. The experiment was continued for 140 minutes during which period a sample was withdrawn regularly. The concentration of HxI was measured in each sample with the results displayed in Table 1. This example shows that at a temperature of 189°C and a flow rate of 33 g/h, a volume of 7 ml of the absorbent prepared as described in Example 1 reduces the hexyl iodide content in an acetic acid stream from above 258 ppb to below 6 ppb. Furthermore, this example shows that the concentration of hexyl iodide in the treated solution is maintained during 140 minutes continuous time on stream.

**Examples 9-12**

The procedure described in Example 8 was repeated except for changes of the absorbent and/or changes in temperature and/or changes in flow rate and/or changes in the hexyl iodide concentration in the untreated acetic acid solution as evident from Table 1. The temperature was varied by varying the set point of the tube furnace and/or by varying the flow rate. For each example the contents of hexyl iodide in the treated samples are also displayed in Table 1. Example 9 demonstrates that also the absorbent prepared as described in Example 2 is efficient in reducing the hexyl iodide concentration at the conditions specified in Table 1. Example 10 shows that the absorbent prepared in Example 1 does not perform as well at a lower temperature and higher flow rate compared to Example 8. However, the hexyl iodide concentration is still strongly reduced compared to the hexyl iodide concentration in the untreated solution. Example 11 clearly demonstrates that at a substantially lower temperature (155°C compared to 189°C in Example 8), the absorbent prepared as described in Example 1 does not perform as well as it does at the higher temperature with respect to decreasing the hexyl iodide concentration. Example 12 demonstrates that with a bed volume of 14 ml (two portions of the absorbent prepared as described in Example 1) instead of a bed volume of 7 ml as used in Example 8, the reduction in hexyl iodide concentration is even larger.

**Example 13**

The procedure described in Example 8 was repeated except that activated charcoal alone was used as the absorbent instead of potassium acetate on activated charcoal. The temperature, the flow rate, the amount of HxI in the untreated solution are recorded in Table 1. For each example, the contents of hexyl iodide in the treated samples are also displayed in Table 1. This example demonstrates that activated charcoal alone is apparently as efficient in decreasing the amount of hexyl iodide in a stream of acetic acid as is activated charcoal impregnated with metal acetate salts. As later examples will show, however, activated charcoal alone will only convert a minor fraction of alkyl iodides to alkyl acetates and the effect of decreasing the level of hexyl iodide in a stream of acetic acid as observed in the present example is primarily a chromtographic effect.

**Example 14**

The procedure described in Example 8 was repeated except that on top of the bed of potassium acetate on activated charcoal (14) was placed another bed (7 ml) of unimpregnated activated charcoal. The temperature, the flow rate, the amount of HxI in the untreated solution are recorded in Table 1. For each example, the contents of hexyl iodide in the treated samples are also displayed in Table 1. This example demonstrates that the amount of hexyl iodide may be reduced to a level below the detection limit of our method (< 0.5 ppb).

**Examples 15-32**

The following examples are all carried out as described in Example 8 with the following deviations: (i) a mixture of methyl iodide, butyl iodide and octyl iodide was used in acetic acid instead of the hexyl iodide/acetic acid mixture (ii) the total concentration of iodide in the untreated solution was in most cases 0.037-0.040 M (37-40 mM) and in some cases 0.0037 M (3.7 mM) (iii) the identity and the amount of the absorbent, the temperature, the flow rate and the specific concentrations of methyl iodide, butyl iodide and octyl iodide were varied as indicated in Table 2. Included in Table 2 for each experiment is the amount of each alkyl iodide and each alkyl acetate measured at between three and five times. For each point in time is calculated how much of the capacity is used of the absorbent bed (Cap (%) in Table 2) calculated as:

$$Cap\ (\%) = (n(MeOAc)+n(BuOAc)+n(OctOAc))/n(M)*100\%$$

where

n (MeOAc) is the total amount of mole methyl acetate formed at the time;
n (BuOAc) is the total amount of mole methyl acetate formed at the time;
n (OctOAc) is the total amount of mole methyl acetate formed at the time;
n (M) is the amount of moles metal in the absorbent as indicated in Table 2.

Similarly, for each point in time is calculated the average conversion of the three alkyl iodides to acetates (Con (%) in Table 2) as:

$$Con\ (\%)= (([MeOAc] / [MeI]\ tot)+([BuOAc]/[BuI]tot)+([OctOAc] / [OctI]tot)\ )/3*100\%,$$

where

[MeOAc] is the measured concentration of methyl acetate in the purified mixture;
[BuOAc] is the measured concentration of butyl acetate in the purified mixture;
[OctOAc] is the measured concentration of octyl acetate in the purified mixture;
[MeI]tot is the concentration of methyl iodide in the unpurified mixture;
[BuI]tot is the concentration of methyl iodide in the unpurified mixture;
[OctI]tot is the concentration of methyl iodide in the unpurified mixture.

Table 1

| | time (min) | ppb HxI | | time (min) | ppb HxI |
|---|---|---|---|---|---|
| Ex **8** 231199A KOAc/A.C. (7 ml) F = 33 ± 8 g/h T = 189 ± 6°C 5 mmole K | --- | 258 | **Ex 12** 091299C KOAc/A.C. (14 ml) F = 43 ± 4 g/h T = 191 ± 4°C 10 mmole K | --- | 130 |
| | 20 | 5.4 | | 20 | < 0.5 |
| | 40 | 2.7 | | 40 | < 0.5 |
| | 60 | 4.0 | | 80 | 0.9 |
| | 120 | 3.2 | | 120 | 1.7 |
| | 140 | 5.9 | | 140 | 2.2 |
| **Ex 9** 241199A CsOAc/A.C. (7 ml) F = 43 ± 4 g/h T = 200 ± 5°C 5 mmole Cs | --- | 258 | **Ex 13** 091299A A.C. (7 ml) F = 42 ± 6 g/h T = 179 ± 2°C 0 mmole metal | --- | 215 |
| | 21 | 3.7 | | 62 | 0.6 |
| | 86 | 5.1 | | 80 | 1.3 |
| | 121 | 3.1 | | 100 | 0.9 |
| | 140 | 3.6 | | 120 | 1.9 |
| | 167 | 4.2 | | 140 | 2.7 |
| **Ex 10** 251199A KOAc/A.C. (7 ml) F = 42 ± 3 g/h T = 183 ± 3°C 5 mmole K | --- | 258 | **Ex 14** 101299A KOAc/A.C. (14 ml) + A.C. (7 ml) F = 41 ± 4 g/h T = 209 ± 2°C 10 mmole K | --- | 109 |
| | 20 | 3.9 | | 41 | < 0.5 |
| | 40 | 5.7 | | 60 | < 0.5 |
| | 80 | 12.4 | | 100 | < 0.5 |
| | 120 | 16.3 | | 120 | < 0.5 |
| | 140 | 7.2 | | 140 | < 0.5 |
| **Ex 11** 261199A KOAc/A.C. (7 ml) F = 38 ± 8 g/h T = 155 ± 3°C 5 mmole K | --- | 258 | | | |
| | 41 | 100 | | | |
| | 60 | 116 | | | |
| | 80 | 93 | | | |
| | 120 | 125 | | | |
| | 140 | 114 | | | |

Table 2

| | time (min) | ppm MeI | ppm MeA | ppm BuI | ppm BuA | ppm OctI | ppm OctA | Cap (%) | Con (%) |
|---|---|---|---|---|---|---|---|---|---|
| **Ex 15** 151099A CsOAc/A.C. (14 ml) F = 29 ± 8 g/h T = 179 ± 3°C 10 mmole Cs [I] = 37.0 mM | --- | 1750 | --- | 2250 | --- | 3000 | --- | --- | --- |
| | 43 | 0 | 509 | 794 | 931 | 0 | 1565 | 6 | 65 |
| | 86 | 0 | 573 | 462 | 1172 | 0 | 1810 | 11 | 76 |
| | 113 | 0 | 572 | 438 | 1161 | 0 | 1680 | 15 | 74 |
| | 123 | 0 | 566 | 444 | 1158 | 0 | 1640 | 16 | 73 |
| | | | | | | | | | |
| **Ex 16** 151099B LiOAc/A.C. (14 ml) F = 35 ± 10 g/h T = 162 ± 5°C 10 mmole Li [I] = 36.8 mM | --- | 1739 | --- | 2236 | --- | 2981 | --- | --- | --- |
| | 41 | 826 | 289 | 1476 | 454 | 0 | 221 | 1 | 25 |
| | 81 | 1187 | 102 | 1948 | 102 | 480 | 922 | 3 | 21 |
| | 102 | 1182 | 65 | 1962 | 65 | 820 | 965 | 4 | 17 |
| | 130 | 1273 | 85 | 1987 | 85 | 722 | 1037 | 6 | 22 |
| | 150 | 1238 | 101 | 1920 | 101 | 559 | 1203 | 7 | 25 |
| **Ex 17** 181099A Zn(OAc)$_2$/A.C. (14 ml) F = 44 ± 3 g/h T = 170 ± 3°C 10 mmole Zn [I] = 36.7 mM | --- | 1739 | --- | 2253 | --- | 2939 | --- | --- | --- |
| | 40 | 940 | 322 | 1123 | 688 | 0 | 238 | 3 | 32 |
| | 81 | 1071 | 294 | 1126 | 719 | 0 | 751 | 7 | 40 |
| | 100 | 1086 | 292 | 1188 | 673 | 0 | 1022 | 10 | 43 |
| | 122 | 1157 | 258 | 1244 | 636 | 0 | 1251 | 12 | 44 |
| | 141 | 1091 | 236 | 1303 | 605 | 0 | 1368 | 15 | 44 |
| **Ex 18** 191099A Mg(OAc)$_2$/A.C. (14 ml) F = 30 ± 14 g/h T = 170 ± 5°C 10 mmole Mg [I] = 37.5 mM | --- | 1775 | --- | 2300 | --- | 3000 | --- | --- | --- |
| | 40 | 1100 | 308 | 1492 | 486 | 0 | 164 | 3 | 25 |
| | 61 | 968 | 403 | 1169 | 695 | 0 | 266 | 4 | 35 |
| | 80 | 946 | 528 | 1125 | 751 | 0 | 396 | 5 | 42 |
| | 125 | 1166 | 276 | 1672 | 413 | 0 | 695 | 7 | 30 |
| | 145 | 1321 | 307 | 1852 | 297 | 91 | 1003 | 9 | 33 |
| **Ex 19** 191099B KOAc/A.C. (14 ml) F = 41 ± 7 g/h T = 179 ± 9°C 10 mmole K [I] = 37.5 mM | --- | 1775 | --- | 2300 | --- | 3000 | --- | --- | --- |
| | 40 | 486 | 560 | 1019 | 727 | 0 | 953 | 4 | 52 |
| | 130 | 619 | 496 | 1167 | 720 | 0 | 1637 | 15 | 60 |
| | 151 | 595 | 510 | 1103 | 695 | 0 | 1928 | 18 | 64 |
| | | | | | | | | | |
| **Ex 20** 201099A A.C. (7 ml) F = 34 ± 3 g/h T = 162 ± 2°C 0 mmole metal [I] = 37.5 mM | --- | 1775 | --- | 2300 | --- | 3000 | --- | --- | --- |
| | 42 | 1324 | 188 | 1514 | 380 | 0 | 0 | --- | 15 |
| | 80 | 1574 | 199 | 1866 | 258 | 0 | 0 | --- | 13 |
| | 102 | 1691 | 207 | 1938 | 134 | 0 | 0 | --- | 11 |
| | 121 | 1775 | 125 | 2059 | 99 | 0 | 34 | --- | 7 |
| | 140 | 1579 | 190 | 2029 | 81 | 0 | 61 | --- | 10 |

Table 2   (continued)

|  | time (min) | ppm MeI | ppm MeA | ppm BuI | ppm BuA | ppm OctI | ppm OctA | Cap (%) | Con (%) |
|---|---|---|---|---|---|---|---|---|---|
| **Exp. 21** 221099A A.C. (7 ml) F = 31 ± 2 g/h T = 172 ± 3°C 0 mmole metal [I] = 37.5 mM | --- | 1775 | --- | 2300 | --- | 3000 | --- | --- | --- |
|  | 43 | 1008 | 286 | 1042 | 710 | 0 | 0 | --- | 27 |
|  | 60 | 1317 | 245 | 1286 | 597 | 0 | 0 | --- | 23 |
|  | 81 | 1372 | 264 | 1536 | 474 | 0 | 0 | --- | 20 |
|  | 119 | 1568 | 311 | 1753 | 335 | 0 | 0 | --- | 19 |
|  | 143 | 1627 | 350 | 1848 | 279 | 0 | 0 | --- | 19 |
| **Ex 22** 281099A KOAc/A.C. (7 ml) F = 44 ± 4 g/h T = 150 ± 1°C 5 mmole K [I] = 39.0 mM | --- | 1791 | --- | 2348 | --- | 3024 | --- | --- | --- |
|  | 80 | 1205 | 243 | 1943 | 146 | 622 | 1346 | 12 | 33 |
|  | 140 | 1261 | 190 | 1996 | 126 | 709 | 1336 | 22 | 30 |
|  | 179 | 1378 | 179 | 2026 | 108 | 739 | 1217 | 29 | 28 |
|  | 220 | 1386 | 144 | 2069 | 101 | 814 | 1229 | 35 | 26 |
|  | 255 | 1411 | 123 | 2091 | 92 | 833 | 1225 | 40 | 25 |
| **Ex 23** 281099D KOAc/A.C. (7 ml) F = 45 ± 3 g/h T = 166 ± 2°C 5 mmole K [I] = 37.9 mM | --- | 1753 | --- | 2351 | --- | 3068 | --- | --- | --- |
|  | 42 | 1019 | 293 | 1801 | 302 | 516 | 1274 | 9 | 37 |
|  | 80 | 1035 | 262 | 1836 | 302 | 492 | 1409 | 17 | 38 |
|  | 100 | 1011 | 275 | 1750 | 338 | 431 | 1468 | 21 | 40 |
|  | 120 | 1029 | 285 | 1756 | 354 | 407 | 1524 | 26 | 41 |
|  | 140 | 1092 | 277 | 1797 | 338 | 424 | 1492 | 31 | 40 |
| **Ex 24** 291099A KOAc/A.C. (7 ml) F = 40 ± 2 g/h T = 194 ± 2°C 5 mmole K [I] = 37.9 mM | --- | 1753 | --- | 2351 | --- | 3068 | --- | --- | --- |
|  | 40 | 437 | 673 | 736 | 929 | 0 | 1119 | 12 | 62 |
|  | 80 | 443 | 761 | 658 | 984 | 0 | 1364 | 26 | 70 |
|  | 101 | 489 | 743 | 703 | 956 | 0 | 1343 | 33 | 69 |
|  | 124 | 536 | 716 | 759 | 927 | 0 | 1307 | 41 | 67 |
|  | 138 | 546 | 693 | 804 | 899 | 0 | 1257 | 46 | 64 |
| **Ex 25** 041199A CsOAc/A.C. (7 ml) F = 35 ± 3 g/h T = 162 + 1°C 5 mmole Cs [I] = 39.9 mM | --- | 2051 | --- | 2350 | --- | 3047 | --- | --- | --- |
|  | 40 | 608 | 701 | 1109 | 776 | 139 | 1614 | 11 | 64 |
|  | 80 | 814 | 607 | 1298 | 679 | 177 | 1904 | 23 | 63 |
|  | 100 | 796 | 602 | 1238 | 716 | 139 | 1896 | 29 | 64 |
|  | 120 | 832 | 594 | 1244 | 730 | 115 | 1800 | 34 | 62 |
|  | 140 | 854 | 577 | 1262 | 709 | 117 | 1758 | 40 | 61 |
| **Ex 26** 041199C Zn(OAc)$_2$/A.C. (7 ml) g F = 41 ± 2 g/h g T = 155 ± 2°C 5 mmole Zn [I] = 39.9 mM | --- | 2051 | --- | 2350 | --- | 3047 | --- | --- | --- |
|  | 60 | 1313 | 249 | 1584 | 503 | 230 | 709 | 8 | 30 |
|  | 80 | 1505 | 147 | 1846 | 320 | 376 | 1215 | 11 | 30 |
|  | 100 | 1598 | 120 | 1935 | 189 | 450 | 1490 | 14 | 31 |
|  | 120 | 1624 | 93 | 1933 | 262 | 474 | 1672 | 18 | 34 |
|  | 140 | 1502 | 0 | 1923 | 233 | 518 | 1573 | 21 | 29 |

Table 2   (continued)

| | time (min) | ppm MeI | ppm MeA | ppm BuI | ppm BuA | ppm OctI | ppm OctA | Cap (%) | Con (%) |
|---|---|---|---|---|---|---|---|---|---|
| **Ex 27** 111199A KOAC/Al$_2$O$_3$ (4 ml) F = 34 ± 3 g/h T = 157 + 2°C 5 mmole K [I] = 39.9 mM g | --- | 2051 | --- | 2350 | --- | 3047 | --- | --- | --- |
| | 40 | 1813 | 0 | 2308 | 0 | 2961 | 155 | 1 | 2 |
| | 80 | 1774 | 0 | 2264 | 0 | 3041 | 65 | 1 | 1 |
| | 100 | 1856 | 0 | 2302 | 0 | 3058 | 40 | 1 | 1 |
| | 126 | 1765 | 0 | 2239 | 0 | 3091 | 32 | 1 | 0 |
| | 145 | 1781 | 0 | 2273 | 0 | 3082 | 31 | 1 | 0 |
| **Ex 28** 111199C KOAc/A.C. (7 ml) F = 45 ± 2 g/h T = 152 ± 2°C 5 mmole K [I] = 3.7 mM | --- | 178 | --- | 230 | --- | 298 | --- | --- | --- |
| | 60 | 117 | 44 | 205 | 34 | 99 | 165 | 2 | 49 |
| | 80 | 121 | 43 | 204 | 0 | 98 | 172 | 2 | 42 |
| | 100 | 115 | 26 | 202 | 37 | 105 | 177 | 2 | 45 |
| | 120 | 121 | 32 | 201 | 0 | 103 | 177 | 3 | 39 |
| | 140 | 131 | 41 | 211 | 0 | 104 | 161 | 3 | 40 |
| **Ex 29** 121199A KOAc/A.C. (7 ml) F = 39 ± 3 g/h T = 174 ± 2°C 5 mmole K [I] = 3.7 mM | --- | 178 | --- | 230 | --- | 298 | --- | --- | --- |
| | 40 | 61 | 77 | 113 | 107 | 0 | 153 | 1 | 76 |
| | 81 | 68 | 83 | 121 | 106 | 15 | 215 | 3 | 88 |
| | 101 | 67 | 52 | 125 | 74 | 20 | 223 | 4 | 70 |
| | 120 | 73 | 77 | 129 | 73 | 20 | 217 | 4 | 78 |
| | 140 | 66 | 73 | 119 | 61 | 20 | 213 | 5 | 73 |
| **Ex 30** 171199A KOAc/A.C. (7 ml) F = 47 ± 3 g/h T = 191 ± 2°C 5 mmole K [I] = 3.7 mM | --- | 178 | --- | 230 | --- | 298 | --- | --- | --- |
| | 40 | 0 | 70 | 42 | 212 | 35 | 117 | 1 | 92 |
| | 80 | 0 | 75 | 83 | 155 | 22 | 209 | 3 | 95 |
| | 100 | 0 | 74 | 55 | 148 | 14 | 226 | 4 | 96 |
| | 120 | 40 | 73 | 88 | 110 | 25 | 231 | 5 | 87 |
| | 140 | 46 | 73 | 108 | 126 | 34 | 240 | 6 | 93 |
| **Ex 31** 181199A KOAC/SiO$_2$ (4 ml) F = 41 ± 2 g/h T = 161 ± 2°C 5 mmole K [I] = 40.0 mM | --- | 2086 | --- | 2404 | --- | 2940 | --- | --- | --- |
| | 40 | 1578 | 110 | 2253 | 0 | 2430 | 242 | 2 | 7 |
| | 80 | 1699 | 95 | 2337 | 0 | 2501 | 214 | 4 | 6 |
| | 100 | 1713 | 89 | 2342 | 0 | 2491 | 202 | 4 | 6 |
| | 120 | 1661 | 79 | 2300 | 0 | 2544 | 197 | 5 | 6 |
| | 140 | 1766 | 77 | 2354 | 0 | 2478 | 183 | 5 | 5 |
| **Ex 32** 181199B CsOAc/A.C. (7 ml) F = 45 ± 1 g/h T = 174 ± 2°C 5 mmole Cs [I] = 40.0 mM | --- | 2086 | --- | 2404 | --- | 2940 | --- | --- | --- |
| | 60 | 341 | 1072 | 555 | 1295 | 0 | 1722 | 32 | 88 |
| | 100 | 429 | 933 | 623 | 864 | 0 | 1543 | 53 | 72 |
| | 120 | 534 | 885 | 720 | 1096 | 38 | 1360 | 62 | 73 |
| | 140 | 656 | 787 | 821 | 1001 | 48 | 1276 | 70 | 66 |
| | | | | | | | | | |

**Claims**

1.  A method for the reduction of iodine content in a process mixture, comprising contacting the organic medium in vapour phase at a temperature from 100°C to 300°C with a metal salt dispersed on activated charcoal.

2.  A method of claim 1, wherein the metal salt is selected from a carboxylate salt, a carbonate, an oxide, a hydroxide and/or a salt being able to react with the organic medium to give a metal carboxylate salt.

3.  A method of claim 1, wherein the metal salt comprises an element selected from a main group element, a lanthanide, an actinide, zinc, copper and silver.

4.  A method as claimed in claim 1, wherein the metal salt comprises an element selected from the main group elements, the lanthanides, the actinides, zinc, copper, alkali metals and alkaline earth metals and combinations thereof.

5.  A method as claimed in claim 1, wherein the metal salt comprises Na, K and/or Cs.

6.  A method as claimed in claim 1, wherein the main component by weight in said mixture is a carboxylic acid and/ or a carboxylic acid anhydride and/or a carboxylic ester and/or an alkylidene dicarboxylate.

7.  A method as claimed in claim 1, wherein the main component by weight in said mixture is acetic acid.

8.  A method according to anyone of the preceding claims, wherein the process mixture comprises carboxylic acids and/or carboxylic anhydrides with alkyl iodides and/or hydriodic acid.